(19) **Europäisches Patentamt**
**European Patent Office**
**Office européen des brevets**

(11) **EP 3 424 560 A1**

(12) **EUROPEAN PATENT APPLICATION**

(43) Date of publication:
**09.01.2019 Bulletin 2019/02**

(51) Int Cl.:
*A61N 5/10* (2006.01)

(21) Application number: **17180044.4**

(22) Date of filing: **06.07.2017**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(71) Applicants:
• **UNIVERSITE CATHOLIQUE DE LOUVAIN**
  **1348 Louvain la Neuve (BE)**
• **Ion Beam Applications S.A.**
  **1348 Louvain-la-Neuve (BE)**

(72) Inventors:
• **ALBERT, Jaroslav**
  **1348 Louvain-la-Neuve (BE)**
• **STERPIN, Edmond**
  **1348 Louvain-la-Neuve (BE)**
• **LABARBE, Rudi Willy R.**
  **1348 Louvain-la-Neuve (BE)**

(74) Representative: **Pecher, Nicolas et al**
**Pecher & Partners**
**Rue Louis de Geer, 6**
**1348 Louvain-la-Neuve (BE)**

(54) **METHOD FOR CORRECTING A CALIBRATION CURVE EXPRESSING THE RELATIONSHIP BETWEEN THE RADIODENSITY AND THE RELATIVE STOPPING POWER OF A HADRON BEAM IN A REGION OF INTEREST**

(57)     The invention relates to a method for correcting at least a portion [H1; H2] of an approximation $S^{rel}(H)$ of a calibration curve, said calibration curve expressing the relationship between a quantitative scale for describing the radiodensity (H) and the relative stopping power for a particle beam, in a region $R$ of interest, characterized in that it comprises the following steps:
- Receiving radiodensity data of said region of interest and an approximation $S^{rel}(H)$ of the calibration curve;
- Receiving a treatment plan comprising a series of N particle beamlets "$i$";
- Computing the WEPL map $W(x, y, z)$;
- Computing the ranges $R_i(x, y)$ for said beamlets of energies $E_i$;
- Assuming that the true WEPL map $W_{true}(x, y, z)$ differs from the computed WEPL map $W(x, y, z)$ by a function $\phi(x, y, z)$ and that

$$\phi'(x, y, z) = \frac{\partial \phi}{\partial z}$$

can be written as a linear combination of basis functions $\xi_j(x, y, z)$:

$$\phi'(x, y, z) = \sum_{j=0}^{M} u_j \xi_j\big(H(x, y, z)\big) \ ;$$

- Computing the expressions

$$\alpha_j(p_i, R) = \left\langle \frac{\int_0^z dz' \xi_j(x_i, y_i, z')}{S^{rel}(x_i, y_i, z)}\bigg|_{z=R} \right\rangle$$

for every beamlet "$i$" where $p_i$ denotes the transverse coordinates $(x_i, y_i)$;
- Measuring with a measuring apparatus the ranges

$$R_m^i$$

of the N beamlets "*i*" ;
- Minimizing the score function

$$D^2 = \frac{1}{N}\sum_{i=1}^{N}\left[R_m^i - \langle R(p_i)\rangle + \sum_{j=0}^{M}u_j\alpha_j(p_i,R)\right]^2$$

with respect to parameters $u_j$ with j= 0...M ;
- Providing the correction to be applied to the approximation of the calibration curve:

$$\phi'(H) = \sum_{j=0}^{M}u_j\xi_j(H);$$

PG profile

Detector

Collimator

$\langle R(p_i)\rangle$

$R_m^i$

Beamlet *i*

Beamlet *i*

**Fig. 1a)**          **Fig. 1b)**          **Fig. 1c)**

**Description**

**Field of the invention**

The present invention relates to a method for correcting a calibration curve, said calibration curve expressing the relationship between a quantitative scale for describing radiodensity (H) and the relative stopping power $S^{rel}(H)$ for a particle beam within a region of interest of a patient undergoing hadron therapy.

The present invention also relates to a system for implementing the method according to the invention.

**Description of prior art**

Hadron therapy, for example proton therapy, for treating a patient has been known for a couple of decades with the prospect of several advantages over conventional radiotherapy. These advantages are due to the physical nature of hadrons. A photon beam in conventional radiotherapy releases its energy according to a decreasing exponential curve as a function of the distance of tissue traversed by the photon beam. By contrast, a hadron beam first releases a small fraction of its energy as it penetrates tissues, forming a plateau. Then, as the hadron path is prolonged, the whole energy is released locally following a steep increase to a peak and a fall-off at the end of the range of the beam. The peak is called Bragg peak and corresponds to the maximum of the Bragg curve. Consequently, a hadron beam can deliver a high dose at a precise location, a target spot, within a target tissue, thus preserving the surrounding healthy tissues. The advantage of hadron therapy allowing the delivery of high doses at a precise location is also one of its weaknesses, because if the position of the Bragg peak of a hadron beam is offset relative to the target tissues, high doses of hadrons may be delivered to adjacent tissues which are healthy. For this reason, the determination of the relative position of the Bragg peak with respect to the position of the target tissue is crucial to properly implement hadron therapy to a patient.

The position of the Bragg peak mainly depends on the initial energy of the hadron beam and on the nature and thicknesses of the traversed tissues. The computation of the initial energy of the hadron beam consequently requires both the determination of the exact position of the target tissue within a patient and also the preliminary characterization of the tissues traversed along the beam path until reaching the target tissue. This characterization is performed during a treatment planning performed before (generally several days before) the actual hadron treatment. The actual hadron treatment can be divided in several sessions distributed over several weeks. A typical treatment plan may start by the acquisition of data, generally in the form of images of the subject of interest with a CT scan. The images acquired by a CT scan yield a 3D representation of a region R comprising the target tissue. In this 3D representation, voxels $(x, y, z)$ are associated with radiodensity data, $H(x,y,z)$, typically in Hounsfield Units (HUs). Once such representation is known, information relating to the energy loss undergone by the hadron beam along its path before reaching the target spot needs to be deduced. Such information will indeed allow determining the right initial energy that the hadron beam must have such that the Bragg peak of the hadron beam is positioned at the right target spot, or in other words has a range corresponding to the target spot.

The energy loss experienced by a hadron beam within a tissue is usually expressed by the hadron stopping power of said tissue, which corresponds to the energy loss of the hadron beam per unit path length within said tissue. Such quantity is usually expressed in relative terms as the hadron stopping power ratio (HSPR), or relative stopping power, and corresponds to the stopping power of the tissue divided by the stopping power of water. It is practical to express the relative stopping power as a function $S^{rel}(H)$ of the radiodensity density data $(H)$. From such function $S^{rel}(H)$, called a calibration curve, and the 3D representation of the radiodensity data $H(x, y, z)$, a map $W(x,y,z)$ representing the water equivalent path length (WEPL) of a hadron beam travelling in the region R can be computed. For a hadron beam travelling in the z-direction, and having transverse coordinates $(x,y)$ in a plane perpendicular to the z-axis, the WEPL map $W(x, y, z)$ is given by the following equation: $W(x,y,z) = \int_{0}^{Z} S^{rel}\big(H(x, y, z')\big)dz'$. $W(x, y, z)$ consequently represents the equivalent distance the hadron beam would travel in water in order to experience to same energy attenuation as when travelling in the region R along the z-axis and with transverse coordinates $(x,y)$. Once such WEPL map $W(x, y, z)$ is computed, the initial energy of a hadron beam required for positioning its Bragg peak at the target spot within region R can easily be deduced from empirical or theoretical relations expressing the position of the Bragg peak of a hadron beam travelling in water and having an given initial energy. An example of relation expressing the range $R_w$, which is the Bragg peak position with respect to its travelling axis, of a hadron beam travelling in water and having an initial energy equal to $E_i$ is for example $R_w = \alpha E_i{}^p$ where $\alpha = 2.2 \times 10^{-3} \left[\frac{cm}{(MeV)^p}\right]$ and $p = 1.77$.

A typical method for obtaining a calibration curve $S^{rel}(H)$ is the so-called stoichiometric calibration. Such method is for example described for protontherapy in «The calibration of CT Hounsfield units for radiotherapy treatment planning» (Schneider U., Pedroni E., Lomax A., Phys. Med. Biol. 1996 ; 41, 111124). In such a method, phantoms whose chemical composition and physical density are known are scanned in a CT scan. The collected radiodensity data are used to deduce the value of constants characterizing scattering processes experienced by X-ray photons when travelling into any medium. From such constants and from assumptions made about the chemical and physical composition of the tissues, both theoretical radiodensity values on one hand and theoretical relative proton stopping powers on the other hand are computed and produce data points, typically one theoretical point per tissue, to which a fit is applied in order to generate the calibration curve $S^{rel}(H)$. Such calibration curve $S^{rel}(H)$ is consequently a generic curve which can be used to establish the treatment plan of a population of patients undergoing protontherapy for irradiating target tissues.

The problem with using this method alone is that radiodensity data obtained from the CT scan only reveals the electronic density in a particular volume of tissues. This is sufficient for photon radiotherapy, but not for proton therapy due to the uncertainty on the information about a quantity known as the mean excitation energy (I), the value of which depending on the molecular composition of the tissue. The tissue mean excitation energy is usually estimated from an approximation of the elemental composition of the tissue as well as on the determination of the "average" value of I for individual elements. In addition, the stoichiometric calibration uses an average tissue composition for a population of patients. However, it is known that for the same type of tissues, there are density differences or differences in water content of tissues between patients. Such individual variability between patients is completely neglected when the same generic curve is applied to all patients receiving a proton therapy treatment.

US 2012/0228493 discusses a more patient-specific recent approach wherein range data from an individual patient are collected via proton radiography. In this prior art, a patient is irradiated with protons of sufficient energy to traverse the patient and their Bragg profiles are detected by a multi-layer ionization chamber or a semiconductor diode. From these profiles, it is possible to tweak the calibration curve in order to be more suitable for the patient. The downside to this approach, however is the fact that the patient must be subjected to additional scanning and hence more radiation dose.

## Summary of the invention

It is an object of the present invention to provide a method for correcting an approximation $S^{rel}(H)$ of a calibration curve expressing the relationship between a quantitative scale for describing radiodensity (H) and the relative stopping power for a particle beam travelling within a region R of interest of a patient undergoing hadron therapy, such that corrected calibration curve is more representative of the individual patient and that it is obtained without exposing the patient to additional radiations.

It is another object of the present invention to provide a system for implementing the method according to the invention.

To this end, the present invention is defined in the appended independent claims.

According to a first aspect, the present invention relates to a method for correcting at least a portion [H1; H2] of an approximation $S^{rel}(H)$ of a calibration curve, said calibration curve expressing the relationship between a quantitative scale for describing the radiodensity (H) and the relative stopping power for a particle beam, in a region R of interest, characterized in that it comprises the following steps:

- Receiving radiodensity data $H(x,y,z)$ for the voxels $(x,y,z)$ of said region R of interest and an approximation $S^{rel}(H)$ of a calibration curve;

- Receiving a treatment plan comprising a series of N particle beamlets "$i$", each beamlet "$i$" being directed parallel to a z-axis and having a lateral spread expressed by a kernel function $K_i(x,y)$ with a standard deviation , said beamlets "$i$" crossing said region R of interest, said beamlets "$i$" being characterized by their initial energy $E_i$ and their transverse coordinates $(x_i,y_i)$ in a plane perpendicular to the z-axis;

- Computing the WEPL ("water equivalent path length") map $W(x, y, z)$ in the z-direction for the voxels $(x,y,z)$ of the region R of interest from the approximation $S^{rel}(H(x,y,z))$ of the calibration curve by using the following equation:

$$W(x, y, z) = \int_0^z S^{rel}\big(H(x, y, z')\big)dz' \; ;$$

- Computing the ranges $\langle R(p_i) \rangle$ for said beamlets of energies $E_i$ along the z-axis in the region R of said beamlets "$i$", where $p_i$ denotes the transverse coordinates $(x_i,y_i)$, and where the notation

$$\langle f(x,y) \rangle = \frac{1}{2\pi\sigma^2} \int_{-\infty}^{\infty} f(x + x', y + y')K_i(x',y')dx'dy'$$ was introduced, by using the WEPL map $W(x, y, z)$

and the following equation :

$$R_W - \int_0^{R_i} S^{rel}\big(H(x,y,z')\big)dz' = 0,$$

wherein $R_w$ is the range in water of a beamlet with an initial energy $E_i$ computed using an empirical or a theoretical formula, and $R_i$ is the range along the $z$-axis in said region R of interest of an infinitely thin beamlet having transverse coordinates equal to $(x,y)$ and with the same initial energy $E_i$ ;

- Assuming that the true WEPL map $W_{true}(x,y,z)$ differs from the computed WEPL map $W(x, y, z)$ by a function $\phi(x,y,z)$ such that $W_{true}(x,y,z) = W(x,y,z)+\phi(x,y,z)$ with $\phi(x,y,z) << W(x,y,z)$ and that $\phi'(x,y,z) = \frac{\partial \phi}{\partial z}$ can be written as a linear combination of (M+1) basis functions $\xi_j(x,y,z)$ wherein said basis functions $\xi_j(x,y,z)$ can be written as functions of $H(x,y,z)$:

$$\phi'(x,y,z) = \sum_{j=0}^{M} u_j \xi_j\big(H(x,y,z)\big) \ ;$$

- Computing the expressions $\alpha_j(p_i, R) = \left\langle \frac{\int_0^z dz' \xi_j(x_i,y_i,z')}{S^{rel}(x_i,y_i,z)}\Big|_{z=R} \right\rangle$ for every beamlet "i";

- Measuring with a range measuring apparatus the ranges $R_m^i$ of the N beamlets "$i$";

- Minimizing the score function $D^2 = \frac{1}{N}\sum_{i=1}^{N}\left[R_m^i - \langle R(p_i)\rangle + \sum_{j=0}^{M} u_j \alpha_j(p_i,R)\right]^2$ with respect to parameters $u_j$ with j= 0...M ;

- Providing the correction to be applied to the approximation of the calibration curve: $\phi'(H) = \sum_{j=0}^{M} u_j \xi_j(H);$

In an advantageous implementation, the N kernel functions $K_i(x,y)$ are Gaussian functions with standard deviation equal to $\sigma_i$ : $e^{\frac{-(x^2+y^2)}{2\sigma_i{}^2}}$ .

In an advantageous implementation, it is assumed that $\phi'(x,y,z) = \frac{\partial \phi}{\partial z}$ can be written as a continuous piecewise linear function, and the step of computing the expressions $\alpha_j(p_i,R)$ comprises the steps of:

- Dividing the portion [H1; H2] of the calibration curve into M segments $[h_j;h_{j+1}]$ with j=0...(M-1) having respectively lengths equal to $L_1 ... L_M$;

- Computing the values of the expressions $\alpha_j(p_i, R) = \left\langle \frac{\int_0^z dz' \xi_j(x_i,y_i,z')}{S^{rel}(x_i,y_i,z)}\Big|_{z=R} \right\rangle$, where the basis functions $\xi_j(x,y,z)$ are the following:

$$\xi_j\big(H(x,y,z)\big) = \left[\frac{h_{j+1}-H}{h_{j+1}-h_j}\Omega_j(H)\big(1-\delta_{jM}\big) + \frac{H-h_{j-1}}{h_j-h_{j-1}}\Omega_{j-1}(H)\big(1-\delta_{j0}\big)\right] ;$$

with $\Omega_j(x) = \begin{cases} 1 \\ 0 \end{cases}$ if $h_{j+1} > x \geq h_j$; otherwise

In a preferred implementation, the method according to claim 3 wherein during the minimizing step the following constraint is imposed on parameters $u_j$ :

$$S^{rel}\left(h_{j+1}\right) + u_{j+1} \geq S^{rel}\left(h_j\right) + u_j.$$

In a preferred implementation, during the minimizing step a maximal relative correction $T$ is imposed on parameters $u_j$ :

$$-T * S^{rel}\left(h_j\right) \leq u_j \leq T * S^{rel}\left(h_j\right).$$

In an advantageous implementation, the radiodensity data $H(x, y, z)$ for the region $R$ of interest are received from computed tomography (CT scan) of the region $R$ of interest.

In an advantageous implementation, the radiodensity data $H(x,y,z)$ for the region R of interest are derived from magnetic resonance imaging (MRI).

In an advantageous implementation, the range measuring apparatus is a prompt gamma camera (PGC).

In an advantageous implementation, the range measuring apparatus is a positron emission tomography (PET) scan.

In an advantageous implementation, the range measuring apparatus is an apparatus configured to perform the range measurements from an ionoacoustic characterization of the proton Bragg peak.

In an advantageous implementation, the range in water $R_w$ of a beamlet with an initial energy $E_i$ is computed using is computed using the Bragg-Kleeman rule.

In an advantageous implementation, the approximation $S^{rel}(H)$ received as input is a calibration curve obtained by stoichiometric calibration and expressing the relationship between a quantitative scale for describing the radiodensity (H) and the relative stopping power for a proton beam.

In an advantageous implementation, the portion [H1; H2] is a portion wherein HU values have a frequency of occurrence higher than a given threshold in the region R of interest

In an advantageous implementation, the steps up to the computation of the expressions $\alpha_j(p_i R)$ are finished before a treatment fraction to patient, said ranges $R_m^i$ of the N beamlets "$i$" being measured during said treatment fraction and said minimizing step being performed during said treatment fraction.

According to a second aspect, the present invention relates to a system for implementing the method according to the invention comprising a range measuring apparatus configured for measuring the ranges $R_m^i$ of the N beamlets "i" and means adapted to execute all the other steps of the method according to the invention.

In an advantageous embodiment, the range measuring apparatus is a prompt gamma camera (PGC).

In an advantageous embodiment, the range measuring apparatus is a positron emission tomography (PET) scan.

According to a third aspect, the present invention relates to a computer program comprising instructions to cause the system according to the invention to execute the steps of the method according to the invention.

**Short description of the drawings**

These and further aspects of the invention will be explained in greater detail by way of example and with reference to the accompanying drawings in which:

**Figure 1** is a schematic representation of the working principle of the method according to the invention;

**Figure 2** shows results obtained for the correction of the calibration curve when using the method according to the invention;

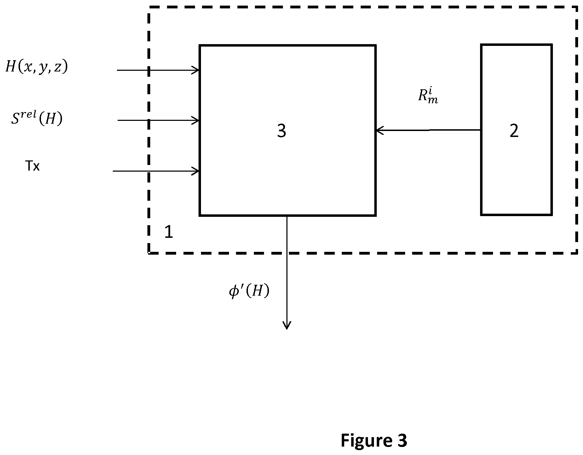

**Figure 3** is a diagram representing a system according to the invention;

**Detailed description of the invention**

The invention relates to a computer implemented method for correcting at least a portion [H1; H2] of an approximation $S^{rel}(H)$ of a calibration curve, said calibration curve expressing the relationship between a quantitative scale for describing the radiodensity (H) and the relative stopping power for a particle beam, in a region R of interest, characterized in that it comprises the following steps:

- Receiving radiodensity data $H(x,y,z)$ for the voxels $(x,y,z)$ of said region R of interest and an approximation $S^{rel}$ $(H)$ of a calibration curve;

- Receiving a treatment plan comprising a series of N particle beamlets "$i$", each beamlet "$i$" being directed parallel to a z-axis and having a lateral spread expressed by a kernel function $K_i(x,y)$, said beamlets "$i$" crossing said region R of interest, said beamlets "$i$" being characterized by their initial energy $E_i$ and their transverse coordinates $(x_i,y_i)$ in a plane perpendicular to the z-axis;

- Computing the WEPL map $W(x,y,z)$ in the z-direction for the voxels $(x,y,z)$ of the region R of interest from the approximation $S^{rel}$ $(H(x,y,z))$ of the calibration curve by using the following equation : $W(x,y,z)$ = $\int_0^z S^{rel}\big(H(x,y,z')\big)dz'$ ;

- Computing the ranges $\langle R(p_i)\rangle$ along the z-axis in the region R of said beamlets "$i$", where $p_i$ denotes the transverse coordinates $(x_i,y_i)$, and where the notation $\langle f(x,y)\rangle = \frac{1}{2\pi\sigma^2}\int_{-\infty}^{\infty} f(x+x',y+y')K_i(x',y')dx'dy'$ was introduced, by using the WEPL map $W(x,y,z)$ and the following equation :

$$R_w - \int_0^{R_i} S^{rel}\big(H(x,y,z')\big)dz' = 0,$$

wherein $R_w$ is the range in water of a beamlet with an initial energy $E_i$ computed using an empirical or a theoretical formula, and $R_i$ is the range along the z-axis in said region R of interest of an infinitely thin beamlet having transverse coordinates equal to $(x,y)$ and with the same initial energy $E_i$ ;

- Assuming that the true WEPL map $W_{true}(x,y,z)$ differs from the computed WEPL map $W(x, y, z)$ by a function $\phi(x,y,z)$ such that $W_{true}(x,y,z) = W(x,y,z)+\phi(x,y,z)$ with $\phi(x,y,z) << W(x,y,z)$ and that $\phi'(x,y,z) = \frac{\partial\phi}{\partial z}$ can be written as a linear combination of (M+1) basis functions $\xi_j(x,y,z)$, wherein said basis functions $\xi_j(x,y,z)$ can be written as functions of $H(x,y,z)$:

$$\phi'(x,y,z) = \sum_{j=0}^M u_j\xi_j\big(H(x,y,z)\big);$$

- Computing the expressions $\alpha_j(p_i,R) = \left\langle \frac{\int_0^z dz'\xi_j(x_i,y_i,z')}{S^{rel}(x_i,y_i,z)}\bigg|_{z=R}\right\rangle$ for every beamlet "$i$";

- Measuring with a range measuring apparatus the ranges $R_m^i$ of the N beamlets "$i$";

- Minimizing the score function $D^2 = \frac{1}{N}\sum_{i=1}^N\big[R_m^i - \langle R(p_i)\rangle + \sum_{j=0}^M u_j\alpha_j(p_i,R)\big]^2$ with respect to parameters $u_j$ with j= 0...M ;

- Providing the correction to be applied to the approximation $S^{rel}$ $(H)$ of the calibration curve: $\phi'(H) = \sum_{j=0}^M u_j\xi_j(H)$ ;

In the above definition of the invention, the portion [H1; H2] is the portion of the quantitative scale for describing the radiodensity (H) wherein the approximation $S^{rel}(H)$ is corrected by the method according to the invention, H1 being the lower bound of the portion and H2 being the upper bound. It is important to note that although the region R is typically a region in the body of a living human patient comprising a target tissue to be irradiated, the method could also be implemented to a region R in any kind of a heterogeneous medium, including non-living materials.

In order to obtain the expressions $\alpha_j(p_i,R) = \left\langle \frac{\int_0^z dz'\xi_j(x_i,y_i,z')}{S^{rel}(x_i,y_i,z)}\bigg|_{z=R}\right\rangle$ provided in the method exposed above, the WEPL map $W_{true}(x,y,z)$ is first expanded in a Taylor series:

$$W_{true}(x,y,R_{true}) = W_{true}(x,y,R) + \frac{\mathrm{d}}{\mathrm{d}z}\,W_{true}(x,y,z)|_{z=R}\,\Delta R + \frac{\mathrm{d}^2}{\mathrm{d}z^2}\,W_{true}(x,y,z)|_{z=R}\,\Delta R^2 + \dots$$

where $\Delta R = R_{true} - R$ is the difference between the range $R_{true}$ which would arise for a given beamlet from a perfect calibration curve and the range R which is computed thanks to the approximation $S^{rel}(H(x,y,z))$ of the calibration curve. If we only take into account the two first terms in the Taylor series, which proves to be a sufficient approximation, we get the following equality:

$$\Delta R(x,y) = R_{true}(x,y) - R(x,y) = \frac{R_w - W_{true}(x,y,z)}{dW_{true}(x,y,z)/dz}\Bigg|_{z=R}$$

As the particle beamlet "$i$" is not infinitely thin but has rather a lateral spread expressed by a kernel function $K_i(x,y)$, with a standard deviation having an order of magnitude of 3mm, the previous equation is advantageously rewritten as follows in order to take this spread into account:

$$\langle \Delta R(x,y) \rangle = \langle R_{true}(x,y) - R(x,y)|_{z=R} \rangle = \left\langle \frac{R_w - W_{true}(x,y,z)}{dW_{true}(x,y,z)/dz}\Bigg|_{z=R} \right\rangle$$

where the notation $\langle f(x,y) \rangle = \frac{1}{2\pi\sigma^2}\int_{-\infty}^{\infty} f(x+x',y+y')K_i(x',y')dx'dy'$ was used.

With the above assumption that $W_{true}(x,y,z) = W(x,y,z) + \phi(x,y,z)$ with $\phi(x,y,z) << W(x,y,z)$, the right hand of the previous equation can be expanded in powers of $\phi$:

$$\left\langle \frac{R_w - W_{true}(x,y,z)}{dW_{true}(x,y,z)/dz} \right\rangle = \left\langle \frac{-\phi}{S^{rel}} \right\rangle + \left\langle \frac{\phi\phi'}{(S^{rel})^2} \right\rangle + \dots$$

where we used the equalities $S^{rel} = dW/dz$ and $W(x,y,R) = R_w$ which can be deduced from the above assumptions, and where we also used the notation $\phi' = \frac{\partial\phi}{\partial z}$. The function $\phi(x,y,z)$ is consequently a correction to the approximation $W(x,y,z)$ of the WEPL map, while $\phi'(x,y,z)$ is a correction to the approximation $S^{rel}(H(x,y,z))$ of the calibration curve. For this reason, $\phi'(x,y,z)$ can also be written as a function of $H(x,y,z)$, which is $\phi'(H(x,y,z)) = \phi'(H)$. Under the assumption that $\phi'(x,y,z)$ can be written as a sum of basis functions $\xi_j(H(x,y,z))$, such that $\phi'(x,y,z) = \sum_{j=0}^{M} u_j\xi_j\big(H(x,y,z)\big)$, with $u_1, u_2, \dots, u_M$ being some parameters, the above equation becomes :

$$\left\langle \frac{R_w - W_{true}(x,y,z)}{dW_{true}(x,y,z)/dz} \right\rangle = -\sum_{j=0}^{M} u_j \left\langle \frac{\int_0^z dz'\xi_j(x,y,z')}{S^{rel}(x,y,z)}\Bigg|_{z=R} \right\rangle + \sum_{j=0}^{M}\sum_{l=0}^{M} u_j u_l \left\langle \frac{\int_0^z dz'\xi_j(x,y,z')\xi_l(x,y,z')}{S^{rel}(x,y,z)}\Bigg|_{z=R} \right\rangle + \dots$$

We consequently see that the expressions $\alpha_j(p_i,R) = \left\langle \frac{\int_0^z dz'\xi_j(x_i,y_i,z')}{S^{rel}(x_i,y_i,z)}\Big|_{z=R} \right\rangle$ correspond to the terms in the first bracket for a beam having its transverse coordinates equal to $p_i = (x_i,y_i)$. The method according to the invention described above is consequently a first-order approximation because only the first term $\left\langle \frac{-\phi}{S^{rel}} \right\rangle$ of the power series is assessed and taken into account in the minimization of the score function $D^2 = \frac{1}{N}\sum_{i=1}^{N}\big[R_m^i -$

$$\langle R(p_i)\rangle + \sum_{j=0}^{M} u_j \alpha_j(p_i, R)\Big]^2$$ with respect to the parameter $u_1, u_2, ..., u_M$.

In other implementations of the method, higher order terms of the power series can be computed and taken into account in the score function. For example, if we take into account the second-order term $\left\langle \frac{\phi\phi'}{(S^{rel})^2} \right\rangle$ of the power series, the score function becomes

$$D^2 = \frac{1}{N} \sum_{i=1}^{N} \Big[ R_m^i - \langle R(p_i)\rangle + \sum_{j=0}^{M} u_j \alpha_j(p_i, R) - \sum_{j=0}^{M} \sum_{l=0}^{M} u_j u_l \, \beta_j(p_i, R) \Big]^2,$$ wherein

$$\beta_j(p_i, R) = \left\langle \frac{\int_0^z dz' \xi_j(x,y,z') \xi_l(x,y,z')}{S^{rel}(x,y,z)} \Big|_{z=R} \right\rangle.$$

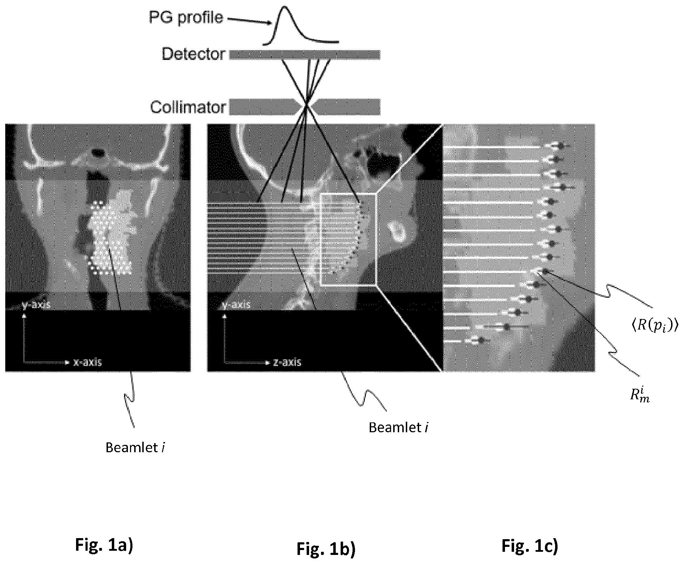

The method according to the invention consequently relies on the minimization of a score function $D^2$ reflecting the discrepancies between the ranges $\langle R(p_i)\rangle$ of N beamlets "$i$" computed from the approximation $S^{rel}(H(x,y,z))$ of the calibration curve and the true ranges $R_m^i$ measured thanks to a measuring apparatus. These true ranges $R_m^i$ correspond to the ranges which would be obtained from a hypothetical true calibration curve. The minimization of the score function $D^2$ yields values for the parameters $u_j$ with j= 0...M which are used to find the correction $\phi'(H)$ to be applied to the approximation $S^{rel}(H)$ of the calibration curve, such to obtain a corrected calibration curve $S^{rel,corr}(H) = S^{rel}(H) + \phi'(H)$. Fig. 1 illustrates the proton beamlets delivered to a patient during a treatment fraction in order to cure a tumor in the neck. In this treatment fraction, a plurality of beamlets having the same initial energy E=133.87 [MeV] are delivered in the neck of a patient. The region R wherein the radiodensity data $H(x,y,z)$ is received is shown in Fig. 1 and each radiodensity value H is represented by a different grey level. In Fig. 1a), the transverse coordinates $(x_i,y_i) = p_i$ are represented in the *xy* transverse plane. In Fig. 1b), the range of the beamlets is represented with respect to the *z*-axis is represented. In Fig. 1c) is an enlarged view of a part of Fig. 1b) wherein both the ranges $\langle R(p_i)\rangle$ and $R_m^i$ of the beamlets are shown in details.

In an advantageous implementation, the range in water $R_w$ of a beamlet with an initial energy $E_i$ is computed using the Bragg-Kleeman rule. Under this assumption, the range $R_w$ is given by the following equation: $R_w = \alpha E_i^{\,p}$ wherein the values of $\alpha$ and $p$ may be obtained by fitting to either ranges or stopping power data from measurements or theory. A judicious choice for the values of these parameters is the following: $\alpha = 2.2 \times 10^{-3} \left[\frac{cm}{(MeV)^p}\right]$ and $p$ = 1.77.

It has to be noted that when the WEPL map $W(x, y, z)$ is computed in the method according to the equation $W(x,y,z) = \int_0^z S^{rel}\big(H(x,y,z')\big) dz'$, the coordinate system wherein the integration is performed is such that the value z = 0 falls in the air upstream the region $R$ of interest, thus ensuring that the z-coordinates of the voxels $(x,y,z)$ in the region $R$ have positive values. The term "upstream" is defined with respect to the direction of the hadron beamlet. The upstream boundary of the region $R$ of interest is typically the skin of a patient. Such choice of coordinate system will guarantee that all the voxels $(x,y,z)$ along the beamlet path in the region $R$ are taken into account when performing the integration of the function $S^{rel}(H(x,y,z'))$ from the value z = 0. If another coordinate system is used, it is then necessary to find an appropriate z value from which the integration can be performed such that all the voxels $(x,y,z)$ along the beamlet path in the region $R$ are taken into account when performing the integration. Furthermore, it is important to note that the energy attenuation of a hadron beamlet in the air before entering the region $R$ of interest is considered as negligible. This involves that the integration can start arbitrarily from any point upstream the boundary of the region $R$ without changing the values of the WEPL map $W(x,y,z)$ in a given coordinate system.

When the ranges $\langle R(p_i)\rangle$ and the expressions $\alpha_j(p_i, R) = \left\langle \frac{\int_0^z dz' \xi_j(x_i,y_i,z')}{S^{rel}(x_i,y_i,z)} \Big|_{z=R} \right\rangle$ are computed in the method according to the invention, convolutions according to the following expression $\langle f(x,y)\rangle = \frac{1}{2\pi\sigma^2} \int_{-\infty}^{\infty} f(x + x', y + y') K_i(x', y') dx' dy'$ need to be assessed at coordinates *(x,y)* equal to the transverse coordinates $(x_i,y_i) = p_i$ of the beamlets "$i$". The kernel function $K_i(x,y)$ describes the lateral spread of the beamlet "$i$" centered at transverse coordinates $(x_i,y_i) = p_i$ and having an energy $E_i$. Such lateral spread of a beamlet "$i$" is machine dependent and consequently depends on the hadron therapy device specifications. Besides, such lateral spread can vary with respect to parameters including the beamlet energy $E_i$ or the orientation of the beam delivery system (gantry angle). This lateral spread can furthermore usually be tuned to some extent by changing some parameters in the hadron therapy device. The kernel function describing the lateral spread of a beamlet "$i$" is typically a Gaussian function $K_i(x,y) = e^{\frac{-(x^2+y^2)}{2\sigma_i^2}}$ . Such Gaussian function has typically a standard deviation $\sigma_i$ having an order of magnitude of 3mm, which depends on the hadron therapy device specifications and tuning, and can vary with respect to parameters including the beamlet energy $E_i$ or the orientation of the beam delivery system (gantry angle). When the lateral spread is a Gaussian function, convolutions according to the following expression $\langle f(x,y)\rangle = \frac{1}{2\pi\sigma^2}\int_{-\infty}^{\infty} f(x+x', y+y')\, e^{\frac{-(x'^2+y'^2)}{2\sigma^2}} dx'dy'$ need to be assessed at coordinates $(x, y)$ equal to the transverse coordinates $(x_i, y_i) = p_i$ of the beamlets "$i$". It has to be noted that even if such convolution involves in theory an integration between $-\infty$ and $+\infty$ for both $x'$ and $y'$, the integration is in practice advantageously numerically assessed over a finite region centered around the transverse coordinates $(x_i, y_i) = p_i$. In practice, it was indeed observed that integrating over a square centered in $(x_i, y_i)$ and having sides of length equal to $6\sigma$ yields sufficiently accurate results for the computation of the ranges $\langle R(p_i)\rangle$ and the expressions $\alpha_j(p_i, R)$. The integration is therefore advantageously performed for values of $(x', y')$ being such that $|x'| \leq 3\sigma$ and $|y'| \leq 3\sigma$, which correspond to a numerical assessment of the following expression: $\langle f(x,y)\rangle = \frac{1}{2\pi\sigma^2}\int_{-3\sigma}^{3\sigma} f(x+x', y+y')e^{\frac{-(x'^2+y'^2)}{2\sigma^2}} dx'dy'$.

In order to implement the method according to the invention, it is then preferred that the region $R$ of interest, for which the radiodensity data $H(x,y,z)$ is received, includes for every beamlet "$i$" a cylinder having its main axis corresponding to the travelling axis of said beamlet "$i$" and having a radius at least equal to $3\sigma$. For the consistence of the range calculations, the initial energy $E_i$ of a beamlet "$i$", which is the energy of the beamlet at the outlet of the beam delivery system, has to correspond to the energy that the beamlet "$i$" has when it enters the region R of interest. The upstream base of the cylinder for which radiodensity data $H(x,y,z)$ is received is consequently preferably located downstream the beam delivery system but upstream any energy attenuating medium found on the path of beamlet "$i$". As the energy attenuation of the hadron beamlet in the air can be considered as negligible, when the beamlet "$i$" is used to cure a patient, this is equivalent to say that the region R for which the radiodensity data $H(x, y, z)$ is received advantageously comprises all the tissues between the outer surface of the patient, usually made of the skin of the patient, and the target tissue wherein the energy of the beam is delivered. The downstream base of the cylinder for which the radiodensity data $H(x,y,z)$ is received is on the other hand advantageously located downstream the range of the beamlet "$i$", such that the cylinder encompasses fully the path of the beamlet in the energy attenuating media. When these conditions for the region R wherein the radiodensity data $H(x, y, z)$ is received are met, the method according to the invention will allow obtaining an optimal correction to the approximation $S^{rel}(H(x,y,z))$ of the calibration curve from the range measurement $R_m^i$ of the beamlet "$i$".

In an advantageous implementation, the radiodensity data $H(x,y,z)$ for every voxel $(x,y,z)$, used in the method according to the invention, is obtained from computed tomography (CT scan) in the region of interest from the patient. In CT imaging, the quantitative scale for describing the radiodensity is usually in Hounsfield units (HU). Such a scale is a linear transformation of the original linear attenuation coefficient into one in which the radiodensity of distilled water at standard pressure and temperature (STP) is defined as zero Hounsfield units, while the radiodensity of air at STP is defined as -1000 HU. In a voxel with average linear attenuation coefficient $\mu$, the corresponding HU is therefore given by:

$$HU = 1000\times \frac{\mu - \mu_{\text{water}}}{\mu_{\text{water}} - \mu_{\text{air}}}$$

where $\mu_{\text{water}}$ and $\mu_{\text{water}}$ are respectively the linear attenuation coefficients of water and air. In this implementation, a preliminary step of computed tomography is performed on the patient in order to get a HU value for every voxel $(x,y,z)$ in the region R of interest.

In another advantageous implementation, the radiodensity data, $H(x,y,z)$, can be derived from MRI data of the region R of interest. Even if MRI does not intrinsically provide radiodensity information directly suitable for replacing CT data, recent methods have indeed been described wherein CT equivalent information is derived from MRI. Such approaches are for example described in « CT substitute derived from MRI sequences with ultrashort echo time » (A.

Johansson, M. Karlsson, T. Nyholm, Medical Physics, Vol. 38, No. 5, May 2011). In this implementation, a preliminary step of MRI is performed on the patient and radiodensitiy data $H(x,y,z)$ is deduced for every voxel $(x,y,z)$ in the region of interest.

In an advantageous implementation, the calibration curve used in the method according to the invention is a curve expressing the relative proton stopping power with respect to radiodensity data. The measuring step of the method according to the invention, during which the range data $R_m^i$ are obtained, is in this case implemented during at least a part of a proton therapy treatment fraction.

A protontherapy treatment plan is indeed generally executed during a treatment phase including one or more treatment sessions, or treatment fractions, during which doses of protons are deposited onto the target tissue. Depending on the pre-established treatment plan, a proton treatment may comprise delivery of a proton beam to a target tissue in various forms, including the following techniques well known in the art: pencil beam/spot scanning, single scattering, double scattering, and uniform scanning/wobbling. The present invention applies to all proton therapy scanning techniques, including pencil beam and uniform scanning techniques. In the present invention, the proton treatment, however, preferably comprises a pencil beam delivery of protons to the target tissue. In this technique, the proton dose delivered to a target spot constitutes a pencil beam, or proton beamlet, and depends on the intensity of the proton beam and on the time of exposure. The proton dose is measured in Grays (Gy), and the dose delivered during a whole treatment fraction is usually of the order of one to several Grays (Gy), while the dose of a single beamlet delivered to a target spot is typically between 0.1 and 20 cGy. During a treatment fraction, a proton beamlet having a first initial energy is directed to a first target spot, during a pre-established delivery time. The proton beamlet is then moved to a second target spot, during a pre-established delivery time. The process is repeated on a sequence of target spots to scan a first iso-energy treatment volume, following a pre-established scanning path. A second iso-energy treatment volume is scanned spot by spot following a similar scanning path with a proton beamlet having a second initial energy. Many iso-energy treatment volumes are usually necessary to treat a given target tissue and are thus irradiated following a similar scanning path. A scanning path can include several passages over a same scanning spot. The iso-energy treatment volumes are thus volumes of target tissues which can be treated with a proton beam having a given initial energy.

In a first protontherapy treatment fraction comprising an irradiation by at least N beamlets "i" parallel to a z-axis, the initial energies of the proton beams are typically derived from a WEPL map computed thanks to an approximation $S^{rel}(H)$ of a calibration curve, which may be a generic calibration curve obtained by stoichiometric calibration. Stoichiometric calibration has been described in the prior art section. During the first protontherapy treatment fraction, the measuring step of the method according to the invention, wherein the ranges $R_m^i$ of the N beamlets "i" are measured, can then be implemented. The range data $R_m^i$ collected during at least a part of the first protontherapy treatment fraction can then be used in the score function minimizing step. Provided the other steps of the method are also implemented, a corrected calibration curve $S^{rel,corr}(H(x,y,z))$ can then be given as output by the method according to the invention. Such corrected calibration curve $S^{rel,corr}(H(x,y,z))$ is a curve describing more accurately the relationship between the radiodensity data and the relative stopping power of the proton beams inside the region R of interest in the patient body. This corrected calibration curve is consequently more representative of the individual patient body than the generic curve, because it is a synthesis between the generic curve $S^{rel}(H(x,y,z))$ and range data $R_m^i$ obtained from proton beams travelling inside the individual patient body. The corrected calibration curve $S^{rel,corr}(H(x,y,z))$ provides thus data reflecting more specifically the chemical composition and the stopping power of the tissues of the individual patient. Such curve consequently offers an insight to the therapists and physicists into the individual variability of patients receiving a protontherapy treatment fraction. With such curve computed from range data $R_m^i$ obtained during at least a part of a first protontherapy treatment fraction, it is in particular possible to compute a new WEPL map $W^{corr}(x,y,z) = \int_0^z S^{rel,corr}\left(H(x,y,z')\right)dz'$ of the region $R$ of interest of the patient. Such new WEPL map can for example be used in order to correct the initial energies of the proton beams delivered in the next phases of the treatment plan, in a next part of the same first treatment fraction or in a next treatment fraction of the treatment plan. The new WEPL map $W^{corr}(x,y,z)$ will indeed allow computing more accurately the ranges of the proton beams travelling in the region R of interest of the patient and consequently the initial energies in the treatment plan that the proton beams must have in order to reach their target spots.

It is important to note that the different steps implemented by the method according to the invention can be performed at different times. Especially the first steps of the method up to and including the step where the expressions $\alpha_j(p_i,R)$ are computed can be performed by a computer once the radiodensity data $H(x,y,z)$ and the approximation $S^{rel}(H)$ of the calibration curve are obtained. All these first steps can consequently be performed before the measurement of the ranges $R_m^i$ during the protontherapy treatment fraction. This can be of interest because the computing steps resulting to the calculation of the expressions $\alpha_j(p_i,R)$ are computationally expensive and consequently take a nonnegligible time. When these steps are performed prior to the treatment fraction, the only remaining steps are the measurement of the ranges $R_m^i$, which needs to be performed during the treatment fraction, and the minimization of the score function

$$D^2 = \frac{1}{N}\sum_{i=1}^{N}\left[R_m^i - \langle R(p_i) \rangle + \sum_{j=0}^{M} u_j \alpha_j(p_i,R)\right]^2$$ with respect to parameters $u_j$ with j= 0...M. These steps only take a time of around a few seconds. Consequently, the splitting of the computational burden between a preliminary phase wherein the expressions $\alpha_j(p_i,R)$ are computed and a second phase wherein the score function $D^2$ is minimized allows getting the corrected calibration function $S^{rel,corr}(H)$ within a very short time after the measurement of the ranges $R_m^i$. This feature of the method according to the invention will make it possible to get the corrected calibration function during a first phase of the treatment fraction and because of the speed of execution it can consequently be used to correct the initial energies of the proton beams delivered to the patient in a second phase of the same treatment fraction.

It is also important to note that although the method according to the invention has been described in previous sections as using range measurements $R_m^i$ from a first fraction of a protontherapy treatment, it can also use range measurements $R_m^i$ obtained during next fractions of the treatment plan. In that case, the method according to the invention can be used in order to apply successive corrections to the calibration curve, by correcting a calibration curve already resulting from an anterior correction obtained from range data of a previous treatment fraction. Such successive corrections making use of successive range data measurements will further increase the accuracy of the resulting corrected calibration curve. The method can also be used to correct the initial approximation $S^{rel}(H)$ of the calibration curve by running the minimization of a score function taking into account ranges $R_m^i$ measured throughout several treatment fractions of a treatment plan. It has to be noted that that the score function $D^2$ can also take into account ranges from beamlets travelling with respect to different nonparallel $z$-axes, delivered for example during different treatment fractions. In this case, a WEPL map $W(x,y,z)$ has to be computed from radiodensity data $H(x,y,z)$ in the region R for every beamlet travelling direction, such that the ranges $\langle R(p_i) \rangle$ of the nonparallel beamlets can all be computed and taken into account in the same score function $D^2$.

It is important to note that everything that was described in the previous sections relating to the application of the method to protontherapy can be applied to other kind of particle therapy, like heavy ion therapy as for example carbon-ion therapy.

The method according to the invention requires thus the presence of a range measuring apparatus in order to measure the ranges $R_m^i$ of the hadron beams crossing the region R of interest. The range measuring apparatus is consequently configured to measure data relating to the hadron beam path in the region R of interest, wherein the measured data allow computing the position of the Bragg peak with respect to the travelling axis of the hadron beam.

In an advantageous embodiment, the range measuring apparatus is a prompt-gamma detector, which is a device for imaging prompt gammas emitted along the hadron beamlet path in the subject of interest. The hadrons of the hadron beamlet can enter in non-elastic collision with atomic nuclei of the tissues traversed. The collision can cause nuclear reaction that lead to the emission of gamma rays, called prompt gammas, through desexcitation. These gamma rays are emitted very quickly (nearly immediately) after the passage of the hadrons.. The gamma prompt camera technique relies on the fact that gamma photons with energies above 1MeV have interesting patterns. They are mainly produced by primary protons along the beam path, emitted isotropically and are not much affected by scattering before leaving the target, so that their fluence is clearly correlated with the beam range. In particular, the 4-5 MeV photons give by far the most interesting contribution (see figure 7e in paper Smeets et al (2012) Prompt gamma imaging with a slit camera for real-time range control in proton therapy. Phys.Med. Biol., 57, 3371-3405). The diminution of the photon fluence at both extremities of the profile is due to the reduced solid angle of the slit opening for photons reaching the scintillator in oblique incidence. The measurements of the prompt gammas yield a prompt gamma profile that can consequently be used to draw conclusions about the Bragg profile of the hadron beamlet and more specifically about the hadron beamlet range. As illustrated in Fig. 1, the prompt gammas can be detected with a prompt gamma camera comprising a collimator and detectors comprising, for example, a scintillator, a photomultiplier and a photon detector. Gamma prompt camera is discussed in detail for example in the following reference: Smeets, J., Roellinghoff, F., Prieels, D., Stichelbaut, F., Benilov, A., Busca, P., ... Dubus, A. (2012). Prompt gamma imaging with a slit camera for real-time range control in proton therapy. Phys.Med. Biol., 57, 3371-3405.

Alternatively, a positron emission tomography (PET) scan can be used. A PET scan is a device for imaging in 3D the concentration of $\beta^+$ (positron) emitter located along the hadron beam path in the subject of interest. A small fraction of the hadrons of the hadron beam create positron emitting isotopes (for example, $^{11}$C, $^{13}$N, $^{15}$O) through interactions with the atomic nuclei of the tissues traversed. These radio-active isotopes decay with emission of a positron which will annihilate with an electron leading to the emission of two gamma photons emitted in coincidence. The PET scan detects the source of emission of these two gamma photons and therefore measures the concentration of $\beta^+$ emitter. The concentration of $\beta^+$ emitter is related to the beam path of the hadron beam. A method to use a PET scan for reconstructing the dose in protontherapy and consequently obtaining range measurements for the proton beams is for example disclosed in "A deconvolution approach for PET-based dose reconstruction in proton radiotherapy" (S. Remmele, J. Hesser, H. Paganetti, T. Bortfeld, Phys. Med. Biol., 56, 2011, 7601-7619).

Alternatively, the range measuring apparatus configured to perform the range measurements from a ionoacoustic characterization of the proton Bragg peak. Such apparatus will detect the thermoacoustic signals that are generated due to localized energy loss of ion beams in tissue. Such kind of technique is for example disclosed in "Ionoacoustic characterization of the proton Bragg peak with submillimeter accuracy" (W. Assmann, S. Kellnberger, S. Reinhardt, S. Lehrack, A. Edlich, P.G. Thirolf, M. Moser, G. Dollinger, M. Omar, V. Ntziachristos, K. Parodi, Medical Physics, 42, 567, 2015).

In an advantageous implementation, it is assumed that the correction

$$\phi'(x,y,z) = \phi'(H(x,y,z)) = \frac{\partial \phi}{\partial z}$$ applied to the approximation $S^{rel}(h_j)$ of the calibration curve can be written as a continuous piecewise linear function, wherein the step of computing the expressions $\alpha_j(p_i,R)$ comprises the following steps:

- Dividing the portion [H1; H2] of the calibration curve into M segments [$h_j;h_{j+1}$] with j=0...(M-1) having respectively lengths equal to $L_1 \dots L_M$;

- Computing the values of the expressions $\alpha_j(p_i, R) = \left\langle \left. \frac{\int_0^z dz' \xi_j(x_i,y_i,z')}{S^{rel}(x_i,y_i,z)} \right|_{z=R} \right\rangle$, where the basis functions $\xi_j(x,y,z)$

are the following:

$$\xi_j\big(H(x,y,z)\big) = \xi_j(H) = \left[ \frac{h_{j+1}-H}{h_{j+1}-h_j} \Omega_j(H)\big(1-\delta_{jM}\big) + \frac{H-h_{j-1}}{h_j-h_{j-1}} \Omega_{j-1}(H)\big(1 - \delta_{j0}\big) \right];$$

with $\Omega_j(x) = \begin{cases} 1 \\ 0 \end{cases}$ if $h_{j+1} > x \geq h_j$; otherwise

Such an implementation wherein the function $\phi'(H)$ is written as a continuous piecewise linear function turns out to be a simple but efficient way to correct the approximation of the calibration curve. The basis functions $\xi_j(H)$ expressed above are in this case triangular functions with a summit in ($h_j$,1), and the two other corners respectively in ($h_{j-1}$,0) and ($h_{j+1}$,0) in the graph of the calibration curve $S^{rel}(H)$. Any linear combination of such overlapping triangular basis functions $\xi_j(H)$ provide a piecewise linear function on the segments [$h_j;h_{j+1}$]. Furthermore, it is interesting to note that in the above expression for $\phi'(H)$, the parameters $u_j$, with j= 0...M, correspond the corrections $\phi'(h_j)$ applied to the radiodensity values $h_j$, with j=0...M, of the portion [H1;H2]. Once the parameters $u_j$ are known from the minimization of the score function $D^2$, the corrected calibration curve $S^{rel,corr}(H)$ can consequently be obtained by applying the corrections $u_j = \phi'(h_j)$ to the values $S^{rel}(h_j)$ of the approximation of the calibration curve, such that $S^{rel,corr}(h_j) = S^{rel}(h_j) + u_j$ and by making the appropriate fit between these graph points.

When the method according to the invention is applied, it is thus necessary to have chosen initially a set of basis functions $\xi_j(H(x,y,z))$ in which the correction $\phi'(x,y,z)$ is decomposed. Besides the implementation provided above, using a piecewise linear assumption for the correction function $\phi'(H)$ and corresponding triangular basis functions $\xi_j(H)$, other implementations wherein other assumptions are made on the shape of $\phi'(H)$ and on the corresponding basis functions $\xi_j(H)$ can be made. Other suitable assumptions on the correction function $\phi'(H)$ would be for example that it is a polynomial function of $H$ or a piecewise polynomial function of $H$, with a given degree $M \geq 2$. In that case, suitable basis functions $\xi_j(H)$ adapted to this kind of assumptions on function $\phi'(H)$ can easily be identified in the mathematical literature.

In an advantageous implementation, constraints can be applied to the computed $u_j$ in order to avoid unrealistic solutions to the minimization problem. For example, it can be imposed that $S^{rel,corr}(H)$ is a monotonically increasing function, or in other terms that $S^{rel,corr}(h_{j+1}) \geq S^{rel,corr}(h_j)$. In the case of a piecewise linear correction, such constraint can be rewritten as $S^{rel}(h_{j+1}) + u_{j+1} \geq S^{rel}(h_j) + u_j$. It can also be imposed that the correction $\phi'(h_j)$ are bounded by in the following range: $-T \times S^{rel}(h_j) \leq \phi'(h_j) \leq T \times S^{rel}(h_j)$. The parameter $T$ is consequently a parameter corresponding to the maximal relative correction that can be applied to the approximation $S^{rel}(H)$ of the calibration curve during the minimization step. In the case of a piecewise linear correction, such constraint can be rewritten as $-T \times S^{rel}(h_j) \leq u_j \leq T \times S^{rel}(h_j)$. Based on experimental evidence, it was found that an appropriate value for the parameter T is 0,05. Choosing such value for T is consequently equivalent to impose that the corrected calibration curve $S^{rel,corr}(H)$ does not differ from the approximation $S^{rel}(H)$ by more than 5% for any value of H in the portion [H1; H2].

In an advantageous implementation, the portion [H1; H2] is portion of the quantitative scale for describing the radiodensity (HU scale) wherein the radiodensity values have a frequency of occurrence higher than a given threshold in the region R of interest. Due to noise observed when measuring the ranges $R_m^i$ of the N beamlets "i" with a range measuring apparatus, like for example a prompt gamma camera (PGC), it turns out that one cannot expect to have a very good correction for the values on the HU scale which have a low frequency of occurrence in the region R of interest. Consequently, it is advantageous to focus the correction of the calibration curve on at least a portion of the HU scale having a frequency of occurrence higher than a given threshold. For a radiodensity scale expresse in HU, such portion is for example equal to the interval [-100 HU; 100 HU].

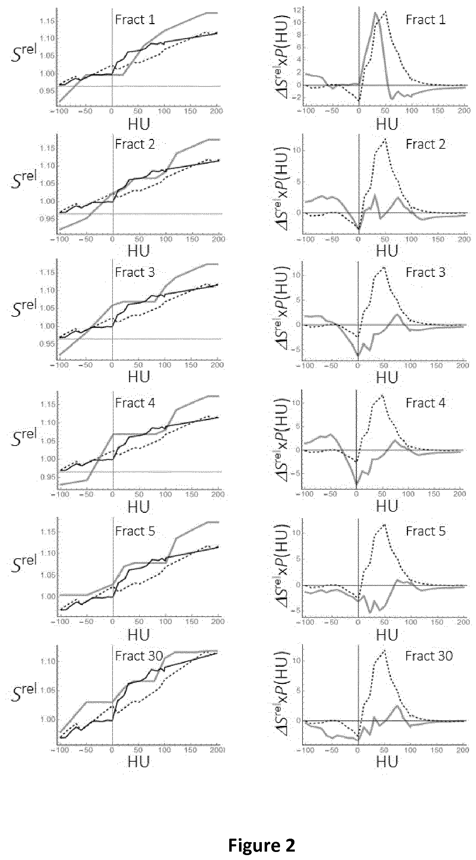

Figure 2 discloses the results obtained by the method according to the invention. In order to assess the reliability of the method, a simulated "true calibration curve" is depicted in black (the darkest curve) in all the left graphs of the figure. Together with a real CT scan image of a region R of interest in a patient body, this simulated "true calibration curve" is consequently used to produce proton beam range data to which "virtual measurements" with a prompt gamma camera (PGC) are applied, by adding noise to the range data at a realistic level expected in such kind of measurements. The generic calibration curve (dashed curves) is also represented on the left graphs. Such generic calibration curve was obtained by stoichiometric calibration and is consequently considered as the calibration curve given as initial input to the method according to the invention. The method according to the invention is indeed used in order to apply successive corrections to the initial calibration curve, by repeating "virtual measurements" with a prompt gamma camera (PGC) and by recomputing at every treatment fraction a correction to the calibration curve obtained from the previous treatment fraction. The successively computed corrected calibration curves $S^{rel,corr}(H)$ are represented in grey in every graph on the left. The different graphs represent the evolution with respect to the number of treatment fractions, after respectively 1, 2, 3, 4, 5 and 30 protontherapy treatment fractions. The graphs on the right depicts on the other hand the difference between respectively the generic calibration curve and the simulated "true calibration curve" (dashed line), and the successively computed corrected calibration curves (grey line), weighted according to the frequency of occurrence of the radiodensity values (HU) in the region R of interest and normalized to 10 000. As it can be noticed in these graphs, after several treatments fractions, the difference between the corrected calibration curves $S^{rel,corr}(H)$ and the simulated "true calibration curve" is significantly lower than the difference between the generic calibration curve and simulated "true calibration curve". This is especially true for the central part of the quantitative scale for describing the radiodensity, between 0 and 100 HU, which correspond to the highest frequency of occurrence for the tissues in a human body.

The invention also relates to a system 1 for implementing the method according to the invention. The system 1 according to the invention comprises a range measuring apparatus 2 for measuring the ranges $R_m^i$ of the N beamlets "i" and means adapted to execute all the other steps of the method claim according to the invention. Such means advantageously comprise computing means 3 configured to receive the input data, including the treatment plan $Tx$, the radiodensity data $H(x, y, z)$ and the approximation $S^{rel}(H)$ of the calibration curve, said computing means 3 being also configured to perform the different computational tasks implemented by the method according to the invention. In an advantageous embodiment, the range measuring apparatus is a prompt gamma camera (PGC). In another advantageous embodiment, the range measuring apparatus is a positron emission tomography (PET) scan. In another advantageous embodiment, the range measuring apparatus is an apparatus configured to perform the range measurements from a ionoacoustic characterization of the proton Bragg peak.

## Claims

1.  Method for correcting at least a portion [H1; H2] of an approximation $S^{rel}(H)$ of a calibration curve, said calibration curve expressing the relationship between a quantitative scale for describing the radiodensity (H) and the relative stopping power for a particle beam, in a region R of interest, **characterized in that** it comprises the following steps:

    - Receiving radiodensity data $H(x,y,z)$ for the voxels $(x,y,z)$ of said region R of interest and an approximation

$S^{rel}(H)$ of a calibration curve;

- Receiving a treatment plan comprising a series of N particle beamlets "$i$", each beamlet "$i$" being directed parallel to a $z$-axis and having a lateral spread expressed by a kernel function $K_i(x,y)$ with a standard deviation , said beamlets "$i$" crossing said region $R$ of interest, said beamlets "$i$" being **characterized by** their initial energy $E_i$ and their transverse coordinates $(x_i, y_i)$ in a plane perpendicular to the $z$-axis;

- Computing the WEPL ("water equivalent path length") map $W(x,y,z)$ in the z-direction for the voxels $(x,y,z)$ of the region R of interest from the approximation $S^{rel}(H(x,y,z))$ of the calibration curve by using the following equation : $W(x, y, z) = \int_0^Z S^{rel}\big(H(x, y, z')\big)dz' \; ;$

- Computing the ranges $\langle R(p_i) \rangle$ for said beamlets of energies $E_i$ along the $z$-axis in the region R of said beamlets "$i$", where $p_i$ denotes the transverse coordinates $(x_i, y_i)$, and where the notation

$\langle f(x,y) \rangle = \frac{1}{2\pi\sigma^2} \int_{-\infty}^{\infty} f(x + x', y + y')K_i(x',y')dx'dy'$ was introduced, by using the WEPL map $W$ (x, y, z) and the following equation :

$$R_w - \int_0^{R_i} S^{rel}\big(H(x, y, z')\big)dz' = 0,$$

wherein $R_w$ is the range in water of a beamlet with an initial energy $E_i$ computed using an empirical or a theoretical formula, and $R_i$ is the range along the $z$-axis in said region R of interest of an infinitely thin beamlet having transverse coordinates equal to $(x,y)$ and with the same initial energy $E_i$ ;

- Assuming that the true WEPL map $W_{true}(x,y,z)$ differs from the computed WEPL map $W$ (x, y, z) by a function $\phi(x,y,z)$ such that $W_{true}(x,y,z) = W(x,y,z) + \phi(x,y,z)$ with $\phi(x,y,z) \ll W$ (x, y, z) and that $\phi'(x, y, z) = \frac{\partial\phi}{\partial z}$ can be written as a linear combination of (M+1) basis functions $\xi_j(x,y,z)$ wherein said basis functions $\xi_j(x,y,z)$ can be written as functions of $H(x,y,z)$:

$$\phi'(x, y, z) = \sum_{j=0}^{M} u_j \xi_j\big(H(x, y, z)\big) \; ;$$

Computing the expressions $\alpha_j(p_i, R) = \left\langle \frac{\int_0^Z dz' \xi_j(x_i, y_i, z')}{S^{rel}(x_i, y_i, z)}\Bigg|_{z=R} \right\rangle$ for every beamlet "i";

- Measuring with a range measuring apparatus the ranges $R_m^i$ of the N beamlets "$i$";

- Minimizing the score function $D^2 = \frac{1}{N} \sum_{i=1}^{N} \left[ R_m^i - \langle R(p_i) \rangle + \sum_{j=0}^{M} u_j \alpha_j(p_i, R) \right]^2$ with respect to parameters $u_j$ with j= 0...M ;

- Providing the correction to be applied to the approximation of the calibration curve:

$\phi'(H) = \sum_{j=0}^{M} u_j \xi_j(H);$

2. Method according to claim 1 wherein the N kernel functions $K_i(x,y)$ are Gaussian functions with standard deviation equal to $\sigma_i$ : $e^{\frac{-(x^2+y^2)}{2\sigma_i^2}}$ .

3. Method according to anyone of the preceding claims, wherein it is assumed that $\phi'(x, y, z) = \frac{\partial\phi}{\partial z}$ can be written as a continuous piecewise linear function, where the step of computing the expressions $\alpha_j(p_i, R)$ comprises the steps of:

- Dividing the portion [H1; H2] of the calibration curve into M segments $[h_j; h_{j+1}]$ with j=0...(M-1) having respectively lengths equal to $L_1 \dots L_M$;

- Computing the values of the expressions $\alpha_j(p_i, R) = \left( \frac{\int_0^Z dz' \xi_j(x_i, y_i, z')}{S^{rel}(x_i, y_i, z)} \Big|_{z=R} \right)$, where the basis functions $\xi_j(x,y,z)$ are the following:

$$\xi_j(H(x,y,z)) = \left[ \frac{h_{j+1}-H}{h_{j+1}-h_j} \Omega_j(H)(1-\delta_{jM}) + \frac{H-h_{j-1}}{h_j-h_{j-1}} \Omega_{j-1}(H)(1-\delta_{j0}) \right];$$

with $\Omega_j(x) = \begin{cases} 1 \\ 0 \end{cases}$ if $h_j+1 > x \geq h_j$; otherwise

4. Method according to claim 3 wherein during the minimizing step the following constraint is imposed on parameters $u_j$ :

$$S^{rel}(h_{j+1}) + u_{j+1} \geq S^{rel}(h_j) + u_j$$

5. Method according to anyone of claims 3 and 4 wherein during the minimizing step a maximal relative correction $T$ is imposed on parameters $u_j$ :

$$-T * S^{rel}(h_j) \leq u_j \leq T * S^{rel}(h_j)$$

6. Method according to anyone of the preceding claims, wherein the range measuring apparatus is a prompt gamma camera (PGC).

7. Method according to anyone of the preceding claims, wherein the range measuring apparatus is a positron emission tomography (PET) scan.

8. Method according to anyone of the preceding claims, wherein the range measuring apparatus is an apparatus configured to perform the range measurements from an ionoacoustic characterization of the proton Bragg peak.

9. Method according to anyone of the preceding claims wherein the range in water $R_w$ of a beamlet with an initial energy $E_i$ is computed using is computed using the Bragg-Kleeman rule.

10. Method according to any one of the preceding claims, wherein the approximation $S^{rel}$ (H) received as input is a calibration curve obtained by stoichiometric calibration and expressing the relationship between a quantitative scale for describing the radiodensity (H) and the relative stopping power for a proton beam.

11. Method according to anyone of the preceding claims, wherein the steps up to the computation of the expressions $\alpha_j(p_i, R)$ are finished before a treatment fraction to patient, said ranges $R_m^i$ of the N beamlets "i" being measured during said treatment fraction and said minimizing step being performed during said treatment fraction.

12. System for implementing the method according to anyone of the preceding claims comprising a range measuring apparatus configured for measuring the ranges $R_m^i$ of the N beamlets "i" and means adapted to execute all the other steps of the method claim according to anyone of the preceding claims.

13. System according to claim 12 wherein the range measuring apparatus is a prompt gamma camera (PGC).

14. System according to claim 12 wherein the range measuring apparatus is a positron emission tomography (PET) scan.

15. Computer program comprising instructions to cause the system of claim 12 to execute the steps of the method of claim 1.

**Fig. 1a)**          **Fig. 1b)**          **Fig. 1c)**

**Figure 2**

$H(x, y, z)$

$S^{rel}(H)$

Tx

$R_m^i$

3

2

1

$\phi'(H)$

**Figure 3**

Europäisches
Patentamt
European
Patent Office
Office européen
des brevets

# EUROPEAN SEARCH REPORT

Application Number

EP 17 18 0044

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | EP 2 229 981 A1 (SCHERRER INST PAUL [CH]) 22 September 2010 (2010-09-22) * paragraph [0032]; claims 1-3 *<br><br>----- | 12-15 | INV.<br>A61N5/10 |
| A | DOOLAN P J ET AL: "Patient-specific stopping power calibration for proton therapy planning based on single-detector proton radiography", PHYSICS IN MEDICINE AND BIOLOGY, INSTITUTE OF PHYSICS PUBLISHING, BRISTOL GB, vol. 60, no. 5, 10 February 2015 (2015-02-10), pages 1901-1917, XP020281343, ISSN: 0031-9155, DOI: 10.1088/0031-9155/60/5/1901 [retrieved on 2015-02-10] * abstract; figure 1 * * chapter 4.5 *<br><br>----- | 12-15 | |

TECHNICAL FIELDS
SEARCHED (IPC)

A61N

The present search report has been drawn up for all claims

| Place of search | Date of completion of the search | Examiner |
|---|---|---|
| Munich | 5 October 2017 | Kajzar, Anna |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 17 18 0044

This annex lists the patent family members relating to the patent documents cited in the above-mentioned European search report.
The members are as contained in the European Patent Office EDP file on
The European Patent Office is in no way liable for these particulars which are merely given for the purpose of information.

05-10-2017

| Patent document cited in search report | | Publication date | Patent family member(s) | | Publication date |
|---|---|---|---|---|---|
| EP 2229981 | A1 | 22-09-2010 | CN | 102438700 A | 02-05-2012 |
| | | | EP | 2229981 A1 | 22-09-2010 |
| | | | EP | 2408523 A1 | 25-01-2012 |
| | | | JP | 5528533 B2 | 25-06-2014 |
| | | | JP | 2012520703 A | 10-09-2012 |
| | | | US | 2012056109 A1 | 08-03-2012 |
| | | | WO | 2010105858 A1 | 23-09-2010 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20120228493 A

**Non-patent literature cited in the description**

- **SCHNEIDER U. ; PEDRONI E. ; LOMAX A.** The calibration of CT Hounsfield units for radiotherapy treatment planning. *Phys. Med. Biol.,* 1996, vol. 41, 111124
- **A. JOHANSSON ; M. KARLSSON ; T. NYHOLM.** CT substitute derived from MRI sequences with ultrashort echo time. *Medical Physics,* May 2011, vol. 38 (5
- **SMEETS et al.** Prompt gamma imaging with a slit camera for real-time range control in proton therapy. *Phys.Med. Biol.,* 2012, vol. 57, 3371-3405
- **SMEETS, J. ; ROELLINGHOFF, F. ; PRIEELS, D. ; STICHELBAUT, F. ; BENILOV, A. ; BUSCA, P. ; DUBUS, A.** Prompt gamma imaging with a slit camera for real-time range control in proton therapy. *Phys.Med. Biol.,* 2012, vol. 57, 3371-3405
- **S. REMMELE ; J. HESSER ; H. PAGANETTI ; T. BORTFELD.** A deconvolution approach for PET-based dose reconstruction in proton radiotherapy. *Phys. Med. Biol.,* 2011, vol. 56, 7601-7619
- **W. ASSMANN ; S. KELLNBERGER ; S. REINHARDT ; S. LEHRACK ; A. EDLICH ; P.G. THIROLF ; M. MOSER ; G. DOLLINGER ; M. OMAR ; V. NTZIACHRISTOS.** Ionoacoustic characterization of the proton Bragg peak with submillimeter accuracy. *Medical Physics,* 2015, vol. 42, 567